# EUROPEAN PATENT APPLICATION

(11) **EP 4 814 987 A1**
(43) Date of publication of application: **30.09.2026**
(21) Application number: 25749100.1
(22) Date of filing: 17.01.2025
(51) Int. Cl.: G16H 50/70, G16H 50/50, G16H 50/20, G16H 30/20, G16H 30/40, A61B 6/50, A61B 6/00, G06T 5/50

(54) **METHOD AND SYSTEM FOR PREDICTING PULMONARY DISEASE ON BASIS OF CHEST RADIOGRAPHIC IMAGE AND LUNG SOUND IMAGE**

(30) Priority: 30.01.2024 KR 20240014295
(71) Applicant: UIF (University Industry Foundation), Yonsei University, Seoul 03722 (KR)
(72) Inventor: LEE, Su Hwan, Seoul 04427 (KR); KIM, Kyu Bom, Seoul 01821 (KR); KIM, Yeon Kyeong, Seoul 03711 (KR)
(74) Representative: HWP Intellectual Property
(86) International application number: PCT/KR2025/000997
(87) International publication number: WO 2025/165013

(57) **Abstract**

An operation method of a pulmonary disease prediction system operated by at least one processor, the operation method includes obtaining a chest radiographic image of a patient and a plurality of lung sound intensity images obtained from at least one chest region of the patient, extracting a lung region from the chest radiographic image to generate a lung region radiographic image , warping the plurality of lung sound intensity images onto the extracted lung region to generate a plurality of lung sound intensity images warped onto the lung region, and obtaining a predicted pulmonary disease prediction result from the lung region radiographic image and the warped plurality of lung sound intensity images, using a pulmonary disease prediction model trained to predict a pulmonary disease from an input image.

## Description

### [Technical Field]

The present disclosure relates to a method and a system for predicting a disease by analyzing medical images based on artificial intelligence.

### [Background Art]

As infectious diseases such as COVID-19 spread, the importance of rapid and accurate diagnosis and prognosis prediction for respiratory and pulmonary diseases is emerging. In general, for the diagnosis and prediction of pulmonary diseases, a method of interpreting a chest radiographic image (or radiograph) and a method of diagnosis using lung sounds through chest auscultation are mainly used.

X-ray-based chest radiography allows for non-invasive observation of the inside of the chest, enabling quantified examination of the lung region, and is widely used in the diagnosis of pulmonary diseases due to relatively low examination costs and rapid examination times. However, if characteristic findings of a pulmonary disease do not clearly appear in the chest radiographic image, difficulties in interpretation may arise due to clinical ambiguity. Accordingly, there is a problem in that additional examinations such as computed tomography (CT) must be performed for an accurate diagnosis, and there may be a risk of radiation exposure due to the nature of chest radiography.

To solve these problems, research is actively being conducted on diagnosing and predicting the prognosis of pulmonary diseases from chest radiographic images using artificial intelligence models. In order to improve the performance of an artificial intelligence model, it is important to effectively learn only the features of the lung region. However, in the related art, since pulmonary disease results are output by taking regions other than the lung region as an input, the accuracy of pulmonary disease prediction may decrease.

Meanwhile, a patient's lung sounds are also one of the important indicators for diagnosing and predicting pulmonary diseases. However, lung sounds are unstructured data in which characteristics of each respiratory disease are not quantified, and when a medical staff member directly auscultates a patient's lung sounds, diagnostic results may vary depending on the medical staff member, or an infection problem of the medical staff member may occur. For this purpose, a non-contact lung sound measurement device is used, but conventional lung sound measurement devices fail to measure lung sounds from a plurality of chest regions, and thus accuracy may be lower compared to an actual auscultation method.

Therefore, a method is required to accurately predict a pulmonary disease based on a patient's chest radiographic image and lung sound data obtained from a plurality of chest regions.

### [Disclosure]

### [Technical Problem]

The present disclosure provides a method and a system for predicting a pulmonary disease based on a chest radiographic image and a lung sound image.

Specifically, the present disclosure is to provide a method and a system for extracting a lung region from a chest radiographic image, warping multi-channel lung sound intensity images obtained over time from a plurality of chest regions onto the extracted lung region radiograph, and predicting a pulmonary disease based on artificial intelligence using the same.

### [Technical Solution]

An embodiment provides an operation method of a pulmonary disease prediction system operated by at least one processor, including: obtaining a chest radiographic image of a patient and a plurality of lung sound intensity images obtained from at least one chest region of the patient, extracting a lung region from the chest radiographic image to generate a lung region radiographic image , warping the plurality of lung sound intensity images onto the extracted lung region to generate a plurality of lung sound intensity images warped onto the lung region, and obtaining a pulmonary disease prediction result from the lung region radiographic image and the warped plurality of lung sound intensity images, using a pulmonary disease prediction model trained to predict a pulmonary disease from an input image.

The lung sound intensity images may be generated based on lung sound signals measured over a predetermined period of time in a plurality of chest regions of the patient using a multi-channel lung sound measurement device.

The lung sound intensity images may be data obtained by expressing a distribution of lung sound intensity, obtained based on the lung sound signals, for each of a plurality of locations at which the lung sounds are measured to generate a lung sound intensity image at a specific point in time, and generating the lung sound intensity image at the specific point in time for each time interval over an entire measurement time.

The generating of the plurality of lung sound intensity images warped onto the lung region may include extracting feature points from the lung region radiographic image and the lung sound intensity images, respectively, and obtaining coordinate values of the respective images based on the feature points; displaying the coordinate values of respective images on a same point map of a two-dimensional plane; calculating a transform matrix between the lung region radiographic image and the lung sound intensity images based on a correspondence relationship obtained by registering the coordinate values of the respective images; and applying the transform matrix to the lung sound intensity images to generate the plurality of lung sound intensity images warped onto the lung region.

The method may further include, prior to the obtaining of the pulmonary disease prediction result, generating training data based on the lung region radiographic image and the warped plurality of lung sound intensity images; and training the pulmonary disease prediction model using the training data.

The generating of the training data may include generating the training data with respect to the lung region radiographic image and the warped plurality of lung sound intensity images by using at least one of a method of concatenating the at least one image, a method of converting the at least one image into color images and blending R, G, and B channels of the converted color images; and a method of concatenating the at least one image with a plurality of domain-transformed images, which are obtained by performing a domain transform on the images.

The obtaining of the pulmonary disease prediction result may further include providing at least one of the lung region radiographic image and the warped plurality of lung sound intensity images, with a region related to the pulmonary disease prediction result distinguishably displayed thereon according to importance.

Another embodiment provides a system for predicting pulmonary disease operated by at least one processor, including: a radiographic image processing unit configured to extract a lung region from a chest radiographic image of a patient to generate a lung region radiographic image ; a warping unit configured to warp a plurality of lung sound intensity images, which are obtained from at least one chest region of the patient, onto the extracted lung region to generate a plurality of lung sound intensity images warped onto the lung region; and a pulmonary disease prediction unit configured to obtain a pulmonary disease prediction result from the lung region radiographic image and the warped plurality of lung sound intensity images, using a pulmonary disease prediction model trained to predict a pulmonary disease from an input image..

The lung sound intensity images may be generated based on lung sound signals measured over a predetermined period of time in a plurality of chest regions of the patient using a multi-channel lung sound measurement device.

The lung sound intensity images may be data obtained by expressing a distribution of lung sound intensity, obtained based on the lung sound signals, for each of a plurality of locations at which the lung sounds are measured to generate a lung sound intensity image at a specific point in time, and generating the lung sound intensity image at the specific point in timefor each time interval over an entire measurement time.

The warping unit may be configured to: extract feature points from the lung region radiographic image and the lung sound intensity images, respectively, and to obtain coordinate values of the respective images based on the feature points; display the coordinate values of the respective images on a same point map of a two-dimensional plane; calculate a transform matrix between the lung region radiographic image and the lung sound intensity images based on a correspondence relationship obtained by registering the coordinate values of the respective images; and apply the transform matrix to the lung sound intensity images to generate the plurality of lung sound intensity images warped onto the lung region.

The pulmonary disease prediction unit may further include a training data generation unit configured to generate training data based on the lung region radiographic image and the warped plurality of lung sound intensity images; and a training unit configured to train the pulmonary disease prediction model using the training data.

The training data generation unit may generate the training data with respect to the lung region radiographic image and the warped plurality of lung sound intensity images by using at least one of a method of concatenating the at least one image, a method of converting the at least one image into color images and blending R, G, and B channels of the converted color images; and a method of concatenating the at least one image with a plurality of domain-transformed images obtained by performing a domain transform on the images.

The system may further include a prediction information providing unit configured to provide at least one of the lung region radiographic image and the warped plurality of lung sound intensity images, with a region related to the pulmonary disease prediction result distinguishably displayed thereon according to importance.

Another embodiment provides a pulmonary disease prediction system, including: a memory storing instructions; and at least one processor configured to execute the instructions, wherein the processor is configured to: extract a lung region from a chest radiographic image to generate a lung region radiographic image ; warp a plurality of lung sound intensity images onto the extracted lung region to generate a plurality of lung sound intensity images warped onto the lung region; and obtain a pulmonary disease prediction result from the lung region radiographic image and the warped plurality of lung sound intensity images, using a pulmonary disease prediction model trained to predict a pulmonary disease from an input image.

The processor may be configured to: extract feature points from the lung region radiographic image and the lung sound intensity images, respectively, to obtain coordinate values of the respective images based on the feature points; display the coordinate values of the respective images on a same point map of a two-dimensional plane; and apply a transform matrix between the lung region radiographic image and the lung sound intensity images, which is calculated based on a correspondence relationship obtained by registering the coordinate values of the respective images, to the lung sound intensity images to generate the plurality of lung sound intensity images warped onto the lung region.

The processor may be configured to generate training data based on the lung region radiographic image and the warped plurality of lung sound intensity images, and train the pulmonary disease prediction model using the training data.

The processor may be configured to generate the training data with respect to the lung region radiographic image and the warped plurality of lung sound intensity images by using at least one of a method of concatenating the at least one image, a method of converting the at least one image into color images and blending R, G, and B channels of the converted color images; and a method of concatenating the at least one image with a plurality of domain-transformed images, which are obtained by performing a domain transform on the images.

The processor may be configured to provide at least one of the lung region radiographic image and the warped plurality of lung sound intensity images, with a region related to the pulmonary disease prediction result distinguishably displayed thereon according to importance.

### [Advantageous Effects]

According to the present disclosure, pulmonary diseases may be predicted more accurately compared to using a single data source by comprehensively analyzing a patient's chest radiographic image and lung sound data.

According to the present disclosure, cohort data in which features of pulmonary diseases are standardized may be secured, thereby allowing the data to be utilized in various medical fields.

According to the present disclosure, conventional chest radiography apparatuses may be utilized, so that the present disclosure may be extensively applied to various medical fields.

According to the present disclosure, information for assisting the medical staff in diagnosis may be provided by visualizing and providing causes of pulmonary diseases by location.

### [Description of the Drawings]

FIG. 1 is a schematic view of a method for predicting respiratory diseases using a pulmonary disease prediction system according to an embodiment.
FIG. 2 is a drawing for explaining a method for generating a lung sound intensity image according to an embodiment.
FIG. 3 is a configuration diagram of a pulmonary disease prediction system according to an embodiment.
FIG. 4 is a drawing for explaining a method for generating a lung sound intensity image warped to a lung region according to an embodiment.
FIG. 5 is a drawing for explaining a pulmonary disease prediction unit according to an embodiment.
FIG. 6 is a drawing for explaining a method for generating various combinations of training data according to an embodiment.
FIG. 7 is a flowchart of a method for predicting a pulmonary disease according to an embodiment.
FIG. 8 is a flowchart of a method for generating a lung sound intensity image warped to a lung region according to an embodiment.
FIG. 9 is a hardware configuration diagram of a pulmonary disease prediction system according to an embodiment.

### [Mode for Invention]

The present disclosure will be described more fully hereinafter with reference to the accompanying drawings, in which embodiments of the disclosure are shown. However, as those skilled in the art would realize, the described embodiments may be modified in various different ways, all without departing from the spirit or scope of the only and present disclosure. Accordingly, the drawings and description are to be regarded as illustrative in nature and not restrictive. Like reference numerals designate like elements throughout the specification.

In the present disclosure, unless explicitly described to the contrary, the word "comprise", and variations such as "comprises" or "comprising", will be understood to imply the inclusion of stated elements but not the exclusion of any other elements. In addition, the terms "-er", "-or", "unit", "portion", "part", and "module" described in the specification mean units for processing at least one function and operation, and can be implemented by hardware components or software components and combinations thereof.

In the present disclosure, apparatuses or devices are configured as hardware including at least one processor, a memory device, a communication device, and the like, and a program executed in combination with the hardware is stored in a designated location. The hardware has a configuration and performance to implement a method of the present disclosure. The program includes instructions that implement the method of operation of the present disclosure described with reference to the drawings, and executes the present disclosure in combination with hardware such as a processor and a memory device.

In the present disclosure, "transmitting or providing" may include not only direct transmission or provision but also indirect transmission or provision through another device or by using a bypass route.

In the the present disclosure, a singular form may be intended to include a plural form as well, unless an explicit expression such as "one" or "single" is used.

In the present disclosure, the same reference numerals refer to the same components regardless of the drawings, and "and/or" includes each and all combinations of one or more of the mentioned components.

In the present disclosure, terms including an ordinal number, such as first, second, and the like, may be used to describe various constituent elements, but the elements are not limited by the terms. The terms are only used to differentiate one constituent element from other constituent elements. For example, a first constituent element could be termed a second constituent element, and similarly, a second constituent element could be termed as a first constituent element, without departing from the scope of the present disclosure.

In the present disclosure, in the flowcharts described with reference to the drawings in this specification, the operation order may be changed, various operations may be merged, certain operations may be divided, and certain operations may not be performed.

In the present disclosure, an artificial intelligence model is an artificial intelligence model that learns at least one task, and may be implemented as a computer program executed on a computing device. The computer program is stored in non-transitory storage media and includes instructions described to execute operations of the present disclosure by a processor. The computer program may be downloaded through a network or sold in the form of a product.

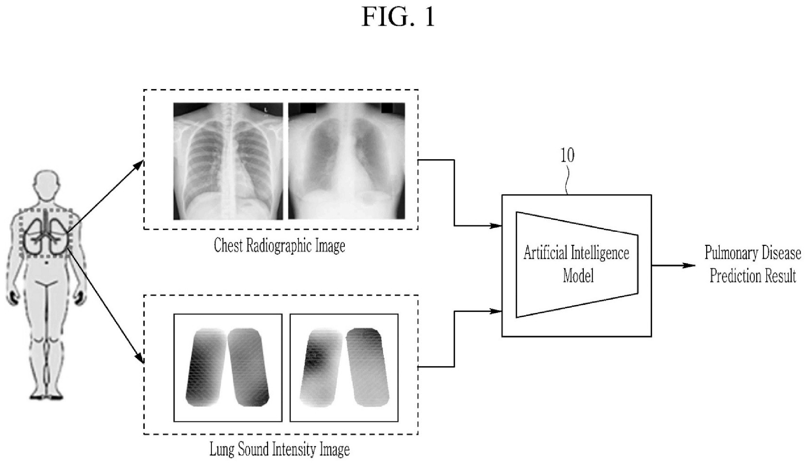

FIG. 1 is a schematic view of a method for predicting respiratory diseases using a pulmonary disease prediction system according to an embodiment.

Referring to FIG. 1, a system 10 for predicting pulmonary disease based on a chest radiographic image and a lung sound image (hereinafter, briefly referred to as a pulmonary disease prediction system 10) is a computing device operating by at least one processor, and may predict pulmonary disease based on artificial intelligence. An artificial intelligence model included in the pulmonary disease prediction system 10 is an artificial intelligence model configured to learn at least one task, and may be implemented in the form of software or a program executed on a computing device.

The pulmonary disease prediction system 10 may receive a chest radiographic image obtained using an X-ray-based chest radiography apparatus and a lung sound intensity image obtained using a multi-channel lung sound measurement system. In this case, the lung sound intensity image may be time-series data representing lung sound intensity according to a measurement position as brightness variations, based on lung sound signals measured in a plurality of chest regions.

The pulmonary disease prediction system 10 may extract a lung region from the chest radiographic image and may warp the lung sound intensity image onto the extracted lung region image.

The pulmonary disease prediction system 10 may train an artificial intelligence model to predict pulmonary disease based on the lung region radiographic image and the lung sound intensity image warped onto the lung region. The pulmonary disease prediction system 10 may predict pulmonary disease using the trained artificial intelligence model. In this case, the artificial intelligence model may be trained and installed by a separate training device, or the pulmonary disease prediction system 10 may generate training data and train the artificial intelligence model based on the training data.

The pulmonary disease prediction system 10 may provide a predicted pulmonary disease result to a hospital information system or an external terminal through a network. In this case, the external terminal may be a smartphone, a PC, or the like, but is not necessarily limited thereto, and may be any device capable of outputting the pulmonary disease result.

Since the pulmonary disease prediction system 10 may comprehensively analyze the patient's chest radiographic image and lung sound signals, it may predict pulmonary disease more accurately than pulmonary disease prediction using single data source. A method by which the pulmonary disease prediction system 10 predicts pulmonary disease will be described in detail below.

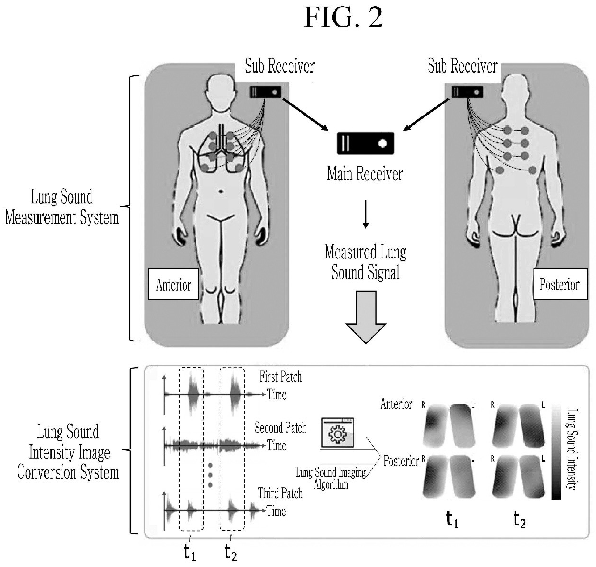

FIG. 2 is a drawing for explaining a method for generating a lung sound intensity image according to an embodiment.

Referring to FIG. 2, the pulmonary disease prediction system 10 may predict pulmonary disease using a plurality of lung sound intensity images obtained based on lung sound signals measured in a plurality of chest regions of a patient. For example, the lung sound signals may be signals measured at an anterior side and a posterior side of the patient's chest using a multi-channel lung sound measurement system, and the number of lung sound measurement regions (the number of patches of the multi-channel lung sound measurement system) may vary according to the judgment of medical staff.

A lung sound intensity image conversion system may generate lung sound intensity images at predetermined time intervals (t₁ to tₙ) for a total lung sound signal in a continuous form. That is, the lung sound intensity image conversion system may repeatedly generate a single lung sound intensity image obtained at a plurality of positions (patches) for a specific time point at predetermined time intervals, and finally generate a plurality of lung sound intensity images in a time-series form.

The pulmonary disease prediction system 10 may obtain the lung sound intensity image generated from the lung sound intensity image conversion system through a network, or obtain a lung sound intensity image stored in an external system such as a hospital information system.

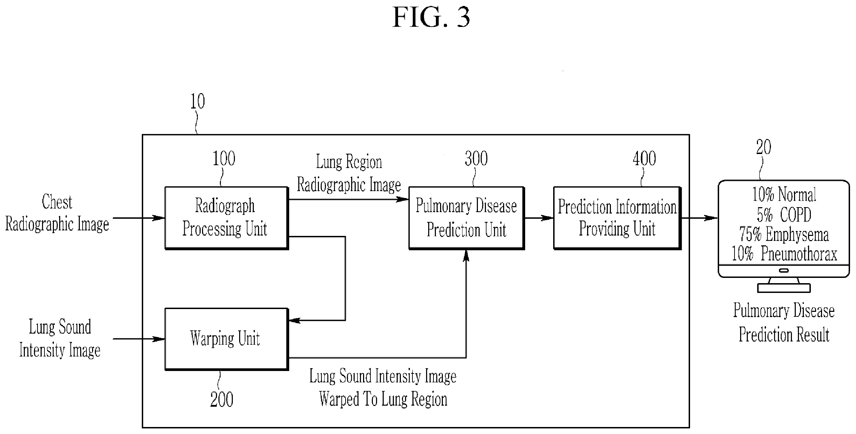

FIG. 3 is a configuration diagram of a pulmonary disease prediction system according to an embodiment.

Referring to FIG. 3, the pulmonary disease prediction system 10 may include a radiograph processing unit 100, a warping unit 200, a pulmonary disease prediction unit 300, and a prediction information providing unit 400 that provides a pulmonary disease prediction result to an external terminal 20.

The radiograph processing unit 100 may receive chest radiographic images capturing an anterior side and a posterior side of a patient's chest. In this case, the radiograph processing unit 100 may perform pre-processing to remove bones, such as a clavicle and a rib, or soft tissues included in the chest radiographic image. For example, the radiograph processing unit 100 may remove bones or soft tissues from the chest radiographic image using a dual-energy-based image processing method. In addition, the radiograph processing unit 100 may use a known image processing method.

The radiograph processing unit 100 may extract a lung region from the chest radiograph to obtain a lung region mask. For example, the radiograph processing unit 100 may extract the lung region according to a probability that pixel values are distributed in an image based on a random walk, or extract the lung region by generating a contour of the lung region using an active contour model. In addition, the radiograph processing unit 100 may extract the lung region using an artificial intelligence model trained to extract a specific region from an image.

The radiograph processing unit 100 may transmit the extracted lung region radiographic image to the warping unit 200 and the pulmonary disease prediction unit 300.

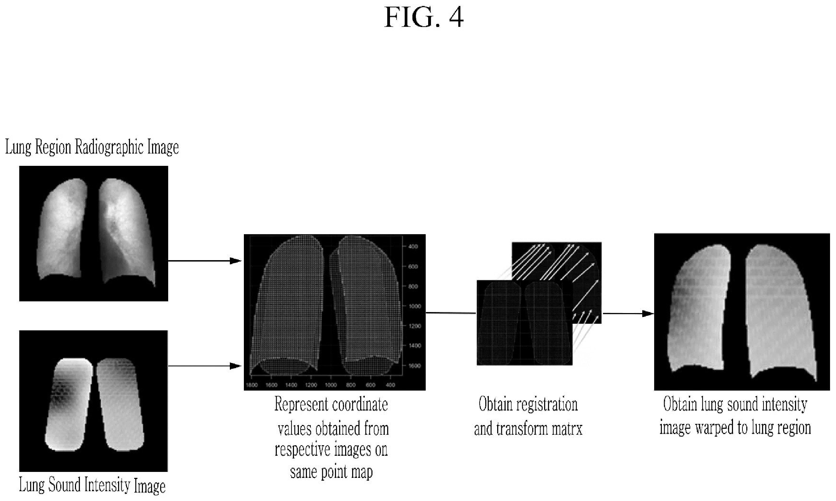

A method by which the warping unit 200 warps the lung sound intensity image onto the lung region will be described in detail with reference to FIG. 4.

FIG. 4 is a drawing for explaining a method for generating a lung sound intensity image warped to a lung region according to an embodiment.

Referring to FIG. 4, the warping unit 200 may receive a chest radiographic image from which a lung region has been extracted from the radiograph processing unit 100, and may warp a lung sound intensity image onto the lung region radiographic image.

The warping unit 200 may respectively extract feature points from the lung region radiographic image and the lung sound intensity image to obtain coordinates corresponding to the lung region or the lung sound intensity image. Thereafter, the warping unit 200 may represent the coordinates obtained from each image on the same point map of a two-dimensional plane using the coordinates.

The warping unit 200 may calculate a transform matrix by registering the coordinates of the lung region radiographic image and the coordinates of the lung sound intensity image represented on the point map. For example, the warping unit 200 may find a correspondence relationship between respective image coordinates using an Iterative Closest Point (ICP) algorithm, and calculate the transform matrix using a Singular Value Decomposition (SVD) algorithm.

The warping unit 200 may calculate the transform matrix including a translation matrix, a shear matrix, a rotation matrix, and a scale matrix, based on the correspondence relationship between the coordinates of the two images found through the registration.

The warping unit 200 may apply the calculated at least one transform matrix to the lung sound intensity image to obtain a lung sound intensity image warped onto the lung region.

Referring back to FIG. 3, the pulmonary disease prediction unit 300 may receive the lung region radiographic image extracted by the radiograph processing unit 100 and the lung sound intensity image warped onto the lung region generated by the warping unit 200.

The pulmonary disease prediction unit 300 may predict a pulmonary disease by extracting features related to a specific pulmonary disease from the received images. In this case, the pulmonary disease prediction unit 300 may include an artificial intelligence model trained to predict the pulmonary disease. For example, the pulmonary disease may be emphysema, chronic obstructive pulmonary disease (COPD), pneumothorax, or the like.

The pulmonary disease prediction unit 300 may visually express on which part of the input lung region radiographic image or the lung sound intensity image warped onto the lung region the pulmonary disease is predicted, by overlaying the information on each image. For example, the pulmonary disease prediction unit 300 may visualize a region related to a pulmonary disease prediction result in the form of a heat map.

The prediction information providing unit 400 may transmit the lung region radiographic image and the lung sound intensity image warped onto the lung region, including a name of the pulmonary disease predicted by the pulmonary disease prediction unit 300, to the external terminal 20, and the external terminal 20 may display the transmitted data on a user interface screen.

The prediction information providing unit 400 may provide stored data to the external terminal 20 according to a request of a user (medical staff). For example, the prediction information providing unit 400 may provide chest radiography information and lung sound measurement information, such as an examination time/period and conditions set during the examination (tube voltage, tube current, etc.), to the external terminal 20.

The prediction information providing unit 400 may provide medical data obtained by interworking with an external system such as a Hospital Information System (HIS) through a network to an external terminal 40.

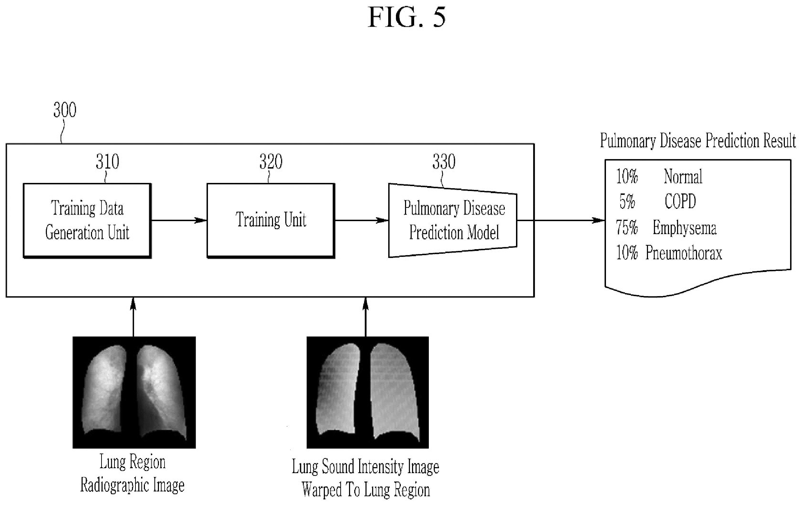

FIG. 5 is a drawing for explaining a pulmonary disease prediction unit according to an embodiment.

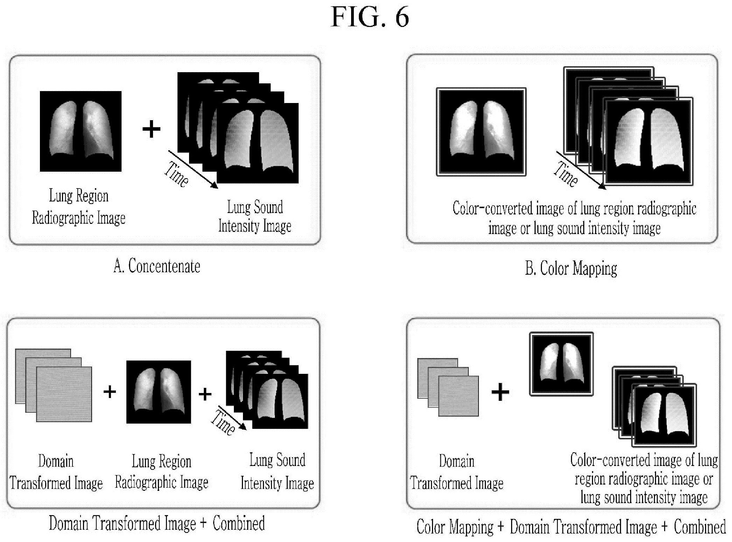

FIG. 6 is a drawing for explaining a method for generating various combinations of training data according to an embodiment.

Referring to FIG. 5, the pulmonary disease prediction unit 300 may include a training data generation unit 310 that generates training data from a lung region radiographic image and a lung sound intensity image warped onto the lung region, and a training unit 320 that trains a pulmonary disease prediction model 330 using the generated training data. In this case, the training unit 320 may not necessarily be included in the pulmonary disease prediction unit 300, but for better understanding and ease of description, the description will be made assuming that the training unit 320 is included in the pulmonary disease prediction unit 300.

Referring to FIG. 6, the training data generation unit 310 may generate a training dataset by combining the lung region radiographic image and the lung sound intensity image in various forms. In this case, the lung sound intensity image may be time-series data generated over a predetermined period of time.

The training data generation unit 310 may generate the training data by concatenating the lung region radiographic image and lung sound intensity images. In this case, the training data generation unit 310 may generate the training data by concatenating only specific images among a plurality of lung sound intensity images.

The training data generation unit 310 may generate the training data by blending the lung region radiographic image and at least one lung sound intensity image, or may convert a grayscale image into a color image having RGB values using a digital image processing (DIP) method and generate training data in a form in which R, G, and B channels of the two images are blended. In this case, the generated training data may be three-dimensional data having R, G, and B values.

The training data generation unit 310 may generate a domain-transformed image for the lung region radiographic image and the at least one lung sound intensity image. For example, the training data generation unit 310 may use a method such as a wavelet transform for domain transformation. The training data generation unit 310 may generate the training data by concatenating the lung region radiographic image, the at least one lung sound intensity image, and the at least one domain-transformed image.

In addition, the training data generation unit 310 may generate the training data by concatenating an image generated by blending after conversion into a color image and the at least one domain-transformed image, among the aforementioned methods.

According to another embodiment, the training data generation unit 310 may generate four-dimensional training data using training data generated by using the aforementioned methods. For example, the training data generation unit 310 may generate the four-dimensional training data by inputting an image generated by blending R, G, and B channels after conversion into a color image among the aforementioned methods together with time information obtained from a time-series lung sound intensity image.

The pulmonary disease prediction model 330 may exhibit improved pulmonary disease prediction performance using various types of training datasets generated by the training data generation unit 310 according to embodiments.

Referring back to FIG. 5, the training unit 320 may receive the training dataset generated by the training data generation unit 310, and train the pulmonary disease prediction model 330 using various combinations of training datasets.

The pulmonary disease prediction model 330 may learn a relationship between the lung region radiographic image, the lung sound intensity image, and the pulmonary disease using the training dataset. That is, the pulmonary disease prediction model 330 may be a deep learning model trained to predict a pulmonary disease from input images. The pulmonary disease prediction model 330 may predict a pulmonary disease such as emphysema, chronic obstructive pulmonary disease (COPD), pneumothorax, or the like, based on the input lung region radiographic image and lung sound intensity image.

The pulmonary disease prediction model 330 may visually represent, on the lung region radiographic image or the lung sound intensity image, the portion of the input image on which the pulmonary disease prediction is based. That is, the pulmonary disease prediction model 330 may be explainable artificial intelligence (XAI), and for this purpose, a method such as a Class Activation Map (CAM) may be applied thereto.

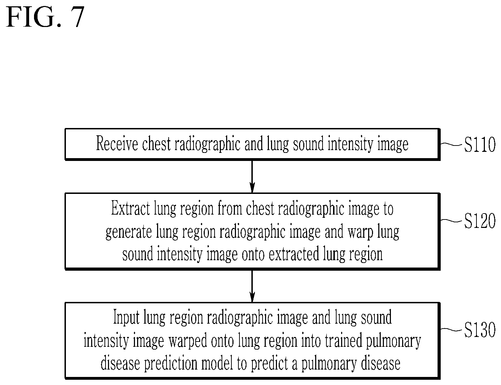

FIG. 7 is a flowchart of a method for predicting a pulmonary disease according to an embodiment.

Referring to FIG. 7, the pulmonary disease prediction system 10 receives a chest radiographic image and a lung sound intensity image (S110). In this case, the chest radiographic image may be a radiographic image of an anterior or posterior view of a patient's chest captured using an X-ray based chest radiography apparatus, and the pulmonary disease prediction system 10 may perform preprocessing to remove bone or soft tissue included in the chest radiographic image. In addition, the lung sound intensity image may be an image representing lung sound intensity by measurement time and measurement position, based on lung sound signals measured in a plurality of chest regions using a multi-channel lung sound measurement system.

The pulmonary disease prediction system 10 extracts a lung region from the chest radiographic image to generate a lung region radiographic image, and warps the lung sound intensity image onto the extracted lung region radigraphic image(S120). The pulmonary disease prediction system 10 may extract the lung region using a random walk, an active contour model, or an artificial intelligence model trained to extract a specific region from an image.

The pulmonary disease prediction system 10 inputs the lung region radiographic image and the lung sound intensity image warped onto the lung region into the trained pulmonary disease prediction model 330 to predict a pulmonary disease (S130). The pulmonary disease prediction model 330 may be a deep learning model trained to predict a pulmonary disease from input images. The pulmonary disease prediction system 10 may train the pulmonary disease prediction model 330 by generating a training dataset in various combinations.

The pulmonary disease prediction system 10 may predict a pulmonary disease such as emphysema, chronic obstructive pulmonary disease (COPD), pneumothorax, or the like, based on the input lung region radiographic image and lung sound intensity image. The pulmonary disease prediction system 10 may provide a predicted pulmonary disease result to a hospital information system or an external terminal through a network.

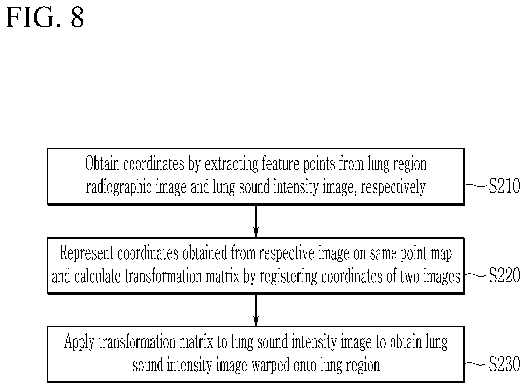

FIG. 8 is a flowchart of a method for generating a lung sound intensity image warped to a lung region according to an embodiment.

Referring to FIG. 8, the pulmonary disease prediction system 10 obtains coordinates by extracting feature points from the lung region radiographic image and the lung sound intensity image, respectively (S210).

The pulmonary disease prediction system 10 represents the coordinates obtained from respective images on the same point map, and calculates a transformation matrix by registering the coordinates of the two images (S220). For example, a correspondence relationship between respective image coordinates may be found using the ICP algorithm, and the transformation matrix may be calculated using the SVD algorithm.

The pulmonary disease prediction system 10 applies the transformation matrix to the lung sound intensity image to obtain the lung sound intensity image warped onto the lung region (S230). The pulmonary disease prediction system 10 may calculate the transform matrix including a translation matrix, a shear matrix, a rotation matrix, and a scale matrix, based on the correspondence relationship between the coordinates of the two images obtained by performing the registration.

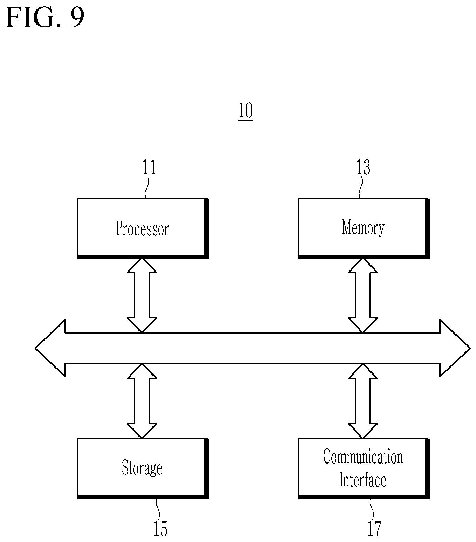

FIG. 9 is a hardware configuration diagram of a pulmonary disease prediction system according to an embodiment.

Referring to FIG. 9, the pulmonary disease prediction system 10 is a computing device operated by one or more processors 11, and may include the processor 11, a memory 13 for loading a computer program executed by the processor 11, a storage 15 for storing the computer program and various data, a communication interface 17, and a bus 19 forconnecting them. In addition, the pulmonary disease prediction system 10 may further include various other components.

The computer program, when loaded into the memory 13, may include instructions that cause the processor 11 to perform methods/operations according to various embodiments of the present disclosure. That is, the processor 11 may perform the methods/operations according to various embodiments of the present disclosure by executing the instructions. The computer program consist of a series of computer-readable instructions grouped based on function and refers to what is executed by a processor. The computer program may include instructions for performing the operations described in the present disclosure.

The processor 11 controls the overall operation of each configuration of the pulmonary disease prediction system 10. The processor 11 may be configured to include at least one of a central processing unit (CPU), a microprocessor unit (MPU), a microcontroller unit (MCU), a graphic processing unit (GPU), or any type of processor well known in the technical field of the present disclosure. In addition, the processor 11 may perform operations for at least one application or computer program for executing methods/operations according to various embodiments of the present disclosure.

The memory 13 stores various data, instructions, and/or information. The memory 13 may load one or more computer programs from the storage 15 to execute methods/operations according to various embodiments of the present disclosure. The memory 13 may be implemented as a volatile memory such as a RAM, but the technical scope of the present disclosure is not limited thereto.

The storage 15 may non-transitorily store the computer program. The storage 15 may be configured to include a non-volatile memory such as a read-only memory (ROM), an erasable programmable ROM (EPROM), an electrically erasable programmable ROM (EEPROM), a flash memory, and the like, a hard disk, a detachable disk, or any form of computer-readable recording medium well known in the technical field to which the present disclosure belongs.

The communication interface 17 supports wired and wireless Internet communication of the pulmonary disease prediction system 10. In addition, the communication interface 17 may support various communication methods other than Internet communication. To this end, the communication interface 17 may be configured to include a communication module well known in the technical field of the present disclosure.

The bus 19 provides a communication function between components of the pulmonary disease prediction system 10. The bus 19 may be implemented as various types of buses, such as an address bus, a data bus, and a control bus.

As described above, according to the present disclosure, since a patient's chest radiographic image and lung sound data may be comprehensively analyzed, a pulmonary disease may be predicted more accurately compared to a single data source.

According to the present disclosure, cohort data in which features of pulmonary diseases are standardized may be secured, thereby allowing the data to be utilized in various medical fields.

According to the present disclosure, conventional chest radiography apparatuses may be utilized, so that the present disclosure may be extensively applied to various medical fields.

According to the present disclosure, information for assisting a diagnosis of medical staff may be provided by visualizing and providing causes of pulmonary diseases by location.

The embodiments of the present disclosure described above are not limited to being implemented only through the apparatus and method, but may also be implemented through a program realizing functions corresponding to the configurations of the embodiments of the present disclosure or a recording medium on which the program is recorded.

While the embodiments of the present disclosure have been described in detail, the scope of the present disclosure is not limited thereto, and various modifications and improvements by a person skilled in the art using the basic concept of the present disclosure as defined in the following claims also belong to the scope of the present disclosure.

## Claims

1. A method of operating a pulmonary disease prediction system operated by at least one processor, the method comprising:
obtaining a chest radiographic image of a patient and a plurality of lung sound intensity images obtained from at least one chest region of the patient;
extracting a lung region from the chest radiographic image to generate a lung region radiographic image ;
warping the plurality of lung sound intensity images onto the extracted lung region to generate a plurality of lung sound intensity images warped onto the lung region; and
obtaining a pulmonary disease prediction result from the lung region radiographic image and the warped plurality of lung sound intensity images, using a pulmonary disease prediction model trained to predict a pulmonary disease from an input image.

2. The method of claim 1, wherein
the lung sound intensity images are generated based on lung sound signals measured over a predetermined period of time in a plurality of chest regions of the patient using a multi-channel lung sound measurement device.

3. The method of claim 2, wherein
the lung sound intensity images are data obtained by expressing a distribution of lung sound intensity, obtained based on the lung sound signals, for each of a plurality of locations at which the lung sounds are measured to generate a lung sound intensity image at a specific point in time, and generating the lung sound intensity image at the specific point in time for each time interbal over an entire measurement time.

4. The method of claim 1, wherein the generating of the plurality of lung sound intensity images warped onto the lung region comprises:
extracting feature points from the lung region radiographic image and the lung sound intensity images, respectively, and obtaining coordinate values of the respective images based on the feature points;
displaying the coordinate values of respective images on a same point map of a two-dimensional plane;
calculating a transform matrix between the lung region radiographic image and the lung sound intensity images based on a correspondence relationship obtained by registering the coordinate values of the respective images; and
applying the transform matrix to the lung sound intensity images to generate the plurality of lung sound intensity images warped onto the lung region.

5. The method of claim 1, further comprising, prior to the obtaining of the pulmonary disease prediction result:
generating training data based on the lung region radiographic image and the warped plurality of lung sound intensity images; and
training the pulmonary disease prediction model using the training data.

6. The method of claim 5, wherein the generating of the training data comprises
generating the training data with respect to the lung region radiographic image and the warped plurality of lung sound intensity images by using at least one of:
a method of concatenating the at least one image;
a method of converting the at least one image into color images and blending R, G, and B channels of the converted color images; and
a method of concatenating the at least one image with a plurality of domain-transformed images obtained by performing a domain transform on the images.

7. The method of claim 1, wherein the obtaining of the pulmonary disease prediction result further comprises
providing at least one of the lung region radiographic image and the warped plurality of lung sound intensity images, with a region related to the pulmonary disease prediction result distinguishably displayed thereon according to importance.

8. A system for predicting pulmonary disease operated by at least one processor, comprising:
a radiograph processing unit configured to extract a lung region from a chest radiographic image of a patient to generate a lung region radiographic image ;
a warping unit configured to warp a plurality of lung sound intensity images, which are obtained from at least one chest region of the patient, onto the extracted lung region to generate a plurality of lung sound intensity images warped onto the lung region; and
a pulmonary disease prediction unit configured to obtain a pulmonary disease prediction result from the lung region radiographic image and the warped plurality of lung sound intensity images, using a pulmonary disease prediction model trained to predict a pulmonary disease from an input image.

9. The system of claim 8, wherein
the lung sound intensity images are generated based on lung sound signals measured over a predetermined period of time in a plurality of chest regions of the patient using a multi-channel lung sound measurement device.

10. The system of claim 9, wherein
the lung sound intensity images are data obtained by expressing a distribution of lung sound intensity, obtained based on the lung sound signals, for each of a plurality of locations at which the lung sounds are measured to generate a lung sound intensity image at a specific point in time, and generating the lung sound intensity image at the specific point in time for each time interval over an entire measurement time.

11. The system of claim 8, wherein the warping unit is configured to:
extract feature points from the lung region radiographic image and the lung sound intensity images, respectively, and to obtain coordinate values of the respective images based on the feature points;
display the coordinate values of the respective images on a same point map of a two-dimensional plane;
calculate a transform matrix between the lung region radiographic image and the lung sound intensity images based on a correspondence relationship obtained by registering the coordinate values of the respective images; and
apply the transform matrix to the lung sound intensity images to generate the plurality of lung sound intensity images warped onto the lung region.

12. The system of claim 8, wherein the pulmonary disease prediction unit further comprises:
a training data generation unit configured to generate training data based on the lung region radiographic image and the warped plurality of lung sound intensity images; and
a training unit configured to train the pulmonary disease prediction model using the training data.

13. The system of claim 12, wherein the training data generation unit is configured to
generates the training data with respect to the lung region radiographic image and the warped plurality of lung sound intensity images by using at least one of:
a method of concatenating the at least one image;
a method of converting the at least one image into color images and blending R, G, and B channels of the converted color images; and
a method of concatenating the at least one image with a plurality of domain-transformed images obtained by performing a domain transform on the images.

14. The system of claim 8, further comprising a prediction information providing unit configured to
provide at least one of the lung region radiographic image and the warped plurality of lung sound intensity images, with a region related to the pulmonary disease prediction result distinguishably displayed thereon according to importance.

15. A pulmonary disease prediction system, comprising:
a memory storing instructions; and
at least one processor configured to execute the instructions,
wherein the processor is configured to:
extract a lung region from a chest radiographic image to generate a lung region radiographic image ;
warp a plurality of lung sound intensity images onto the extracted lung region to generate a plurality of lung sound intensity images warped onto the lung region; and
obtain a pulmonary disease prediction result from the lung region radiographic image and the warped plurality of lung sound intensity images, using a pulmonary disease prediction model trained to predict a pulmonary disease from an input image.

16. The system of claim 15, wherein the processor is configured to:
extract feature points from the lung region radiographic image and the lung sound intensity images, respectively, to obtain coordinate values of the respective images based on the feature points;
display the coordinate values of the respective images on a same point map of a two-dimensional plane; and
apply a transform matrix between the lung region radiographic image and the lung sound intensity images, which is calculated based on a correspondence relationship obtained by registering the coordinate values of the respective images, to the lung sound intensity images to generate the plurality of lung sound intensity images warped onto the lung region.

17. The system of claim 15, wherein the processor is configured to
generate training data based on the lung region radiographic image and the warped plurality of lung sound intensity images, and train the pulmonary disease prediction model using the training data.

18. The system of claim 17, wherein the processor is configured to
generate the training data with respect to the lung region radiographic image and the warped plurality of lung sound intensity images by using at least one of:
a method of concatenating the at least one image;
a method of converting the at least one image into color images and blending R, G, and B channels of the converted color images; and
a method of concatenating the at least one image with a plurality of domain-transformed images, which are obtained by performing a domain transform on the images.

19. The system of claim 15, wherein the processor is configured to
provide at least one of the lung region radiographic image and the warped plurality of lung sound intensity images, with a region related to the pulmonary disease prediction result distinguishably displayed thereon according to importance.
